# EUROPEAN PATENT SPECIFICATION

(11) **EP 4 017 361 B1**
(45) Date of publication and mention of the grant of the patent: **18.06.2025**
(21) Application number: 20854350.4
(22) Date of filing: 20.02.2020
(51) Int. Cl.: A61B 5/145, A61B 5/1473

(54) **MICRO-ANALYTE DETECTION DEVICE**
MIKROANALYTDETEKTOR
DISPOSITIF DE DÉTECTION DE MICRO-ANALYTE

(30) Priority: 19.08.2019 WO PCT/CN2019/101271
(43) Date of publication of application: 29.06.2022
(73) Proprietor: Medtrum Technologies Inc., Shanghai 201203 (CN)
(72) Inventor: YANG, Cuijun, Shanghai 201203 (CN)
(74) Representative: Becker, Eberhard
(86) International application number: PCT/CN2020/075969
(87) International publication number: WO 2021/031542

(56) References cited:
- WO-A1-2010/091005
- WO-A1-2015/131432
- WO-A1-2018/027940
- CN-A- 103 750 819
- CN-A- 104 739 444
- CN-A- 104 887 242
- CN-A- 106 137 214
- CN-A- 107 014 877
- CN-A- 107 949 314
- CN-A- 108 471 959
- CN-A- 109 310 373
- CN-A- 109 998 555
- CN-A- 110 584 676
- CN-U- 204 542 144
- US-A1- 2006 020 191
- US-A1- 2010 217 093
- US-A1- 2015 190 075

## Description

### TECHNICAL FIELD

The present invention mainly relates to the field of medical device, and in particular, to a micro-analyte detection device.

### BACKGROUND

The pancreas in a normal person can automatically monitor the amount of glucose in the blood and automatically secrete the required dosage of insulin/glucagon. However, for diabetic patients, the function of the pancreas is abnormal, and the pancreas cannot normally secrete required dosage of insulin. Therefore, diabetes is a metabolic disease caused by abnormal pancreatic function, which is also a lifelong disease. At present, medical technology cannot cure diabetes, and it can only control the occurrence and development of diabetes and its complications by stabilizing blood glucose.

Patients with diabetes need to check their blood glucose before injecting insulin into the body. At present, most of the detection methods can continuously detect blood glucose, and send the blood glucose data to the remote device in real time for the user to view. This detection method is called Continuous Glucose Monitoring (CGM) method. The method requires the sensing device to be attached to the surface of the patients' skin, and the probe carried by the device is inserted into the subcutaneous tissue fluid for testing.

However, the current detecting device is relatively thick, affecting the user's dressing, stretching, exercise and other daily activities, which can seriously worsen user experience. Also, detection can be easily interrupted with such a sensing device because a bulky device can get bumped or caught easily, which may lead to data loss and pose a potential safety hazard to the user.

Accordingly, there is a need in the state of the art for a detection device that has a reduced thickness and enhances the user experience.

US 7946984 B2 relates to systems and methods for measuring an analyte in a host for transcutaneous measurement of glucose in a host. WO 2015/131432 A1 relates to an analyte sensing system which uses an automatic installer to implant a sensor probe into a human body and obtains analyte content information collected by the sensor probe through a transmitter and a receiver. WO 2010/091005 A1 relates to an apparatus for insertion of a medical device in the skin of a subject.

### BRIEF SUMMARY OF THE INVENTION

The invention is set out in the appended set of claims. The following disclosure serves a better understanding of the present invention. The embodiment of the invention discloses a micro-analyte detection device. The sensor structure is embedded in the bottom board of the bottom case, and the distance between the top of the sensor structure and the inner bottom surface of the bottom case is small. There is no need to provide extra space in the transmitter to accommodate the sensor structure portion that protrudes the inner bottom surface of the bottom case, which reduces the overall thickness of the analyte detection device and enhances the user experience.

The invention relates to a micro-analyte detection device, which includes a bottom case, a bottom board of the bottom case is provided with a mounting hole, and a first fastener part is provided at an edge of the mounting hole; a sensor structure, the sensor structure includes a sensor and a sensor base, and the sensor includes a signal output end and a detection end, a second fastener part is provided at an edge of the sensor base, contour shapes of the edge of the sensor base and that of the mounting hole match each other, the first fastener part and the second fastener part are fastened with each other, the edge of the mounting hole and the edge of the sensor base are interlocked, and the sensor base is installed in the mounting hole; and a transmitter, the transmitter includes a transmitter housing and an electronic device disposed in the transmitter housing, the electronic device is provided with an electrical connection area, when the transmitter is installed in a working position, the electrical connection area is electrically connected with the signal output end.

According to one aspect of the present invention, the sensor's shape is an arc or a polyline.

According to one aspect of the present invention, the signal output end is a planar structure and is laid on a top of the sensor base.

According to one aspect of the present invention, a straight line determined by a longitudinal direction of the signal output end and a straight line determined by a longitudinal direction of the detection end intersect or are in different planes.

According to one aspect of the present invention, the straight line determined by the longitudinal direction of the signal output end is perpendicular to the straight line determined by the longitudinal direction of the detection end.

According to one aspect of the present invention, the straight line determined by the longitudinal direction of the detection end is perpendicular to a skin surface where the detection end punctures.

According to one aspect of the present invention, the electrical connection area is exposed outside the transmitter housing, and a position of the electrical connection area corresponds to a position of the signal output end.

According to one aspect of the present invention, further includes a conductive rubber strip, which is disposed between the electrical connection area and the signal output end, and the electrical connection area and the signal output end are electrically connected through the conductive rubber strip.

According to one aspect of the present invention, further includes a sealing member, and the sealing member is arranged around the position where a signal output end is electrically connected with the electrical connection area.

Compared with the prior art, the technical solution of the present invention has the following advantages:
The bottom board of the bottom case of the micro-analyte detection device disclosed by the present invention is provided with a mounting hole, and a first fastener part is provided at the edge of the mounting hole. The mounting hole encircles the entire sensor base. A second fastener part is provided at the edge of the sensor base of the sensor structure, and the contour shapes of the edge of the sensor base and of the mounting hole match each other, the first fastener part and the second fastener part are fastened with each other, the edge of the mounting hole and the edge of the sensor base are interlocked. The matching contour shapes of the sensor base and the mounting hole ensure that the sensor base can be completely embedded in the bottom board of the bottom case, making the top of the sensor structure not higher than or slightly higher than the inner bottom surface of the bottom case. At the same time, the first fastener part and the second fastener part are respectively disposed on the edge of the mounting hole and the edge of the sensor base, so that the top of the fastener position is also not higher than or slightly higher than the inner bottom surface of the bottom case. This design greatly reduces the height of the sensor structure protruding from the inner bottom surface of the bottom case. Therefore, no extra space is required along the thickness direction of the transmitter to accommodate the foresaid protruding portion, which reduces the thickness of the analyte detection device and enhances user experience.

Furthermore, the sensor's shape is an arc or a polyline instead of a straight line. A sensor shape of arc or polyline can further reduce the height of the sensor structure, which further reduces the thickness of the analyte detection device.

Furthermore, the signal output end is a planar structure and is laid on the top of the sensor base. The planar structure of the signal output end facilitates its electrical connection with the electrical connection area of the transmitter. In addition, the flat signal output end is laid on the top of the sensor base, which further reduces the overall height of the sensor structure and, as a result, further reduces the thickness of the analyte detection device.

Furthermore, the straight line determined by the longitudinal direction of the signal output end is perpendicular to the straight line determined by the longitudinal direction of the detection end. That the two straight lines are perpendicular to each other can optimize the design of the installation equipment of the sensor structure, which will simplify the installation process of the sensor structure and facilitate the puncture process.

Furthermore, the conductive rubber strip is disposed between the electrical connection area and the signal output end, and the electrical connection area and the signal output end are electrically connected through the conductive rubber strip. The electrical connection area is electrically connected to the signal output end by pressing the elastic conductive rubber strip to ensure good electrical connection between these two parts.

### BRIEF DESCRIPTION OF THE DRAWINGS

FIG.1 is a schematic perspective view of a bottom case according to an embodiment of the present invention;
FIG.2 is a schematic perspective view of a sensor structure according to an embodiment of the present invention;
FIG.3 is a schematic perspective view of a sealing member and a sensor structure according to an embodiment of the present invention;
FIG.4a and FIG.4b are schematic perspective views of a bottom case, a sensor structure and a sealing member before and after assembly according to an embodiment of the present invention, respectively;
FIG.4c is a schematic sectional view of a part of the longitudinal section of FIG.4b;
FIG.5a is a schematic perspective view of a sensor structure with a sealing member installed according to an embodiment of the present invention;
FIG.5b and FIG.5c are schematic sectional views of longitudinal sections of a sensor and its adjacent structures obtained along a section line A-A' in FIG.5a;
FIG.5d is a schematic perspective view of a sensor according to an embodiment of the present invention;
FIG.6 is a schematic sectional view of a part of the longitudinal section of a sensor according to another embodiment of the present invention;
FIG.7 is a schematic perspective view of a transmitter according to an embodiment of the present invention;
FIG.8a is a schematic perspective view of a bottom case, a sensor structure, a sealing member and a transmitter according to an embodiment of the present invention;
FIG.8b is a side view of a micro-analyte detection device according to an embodiment of the present invention;
FIG.8c is a schematic sectional view of the sensor in FIG.8b and its positional relationship with adjacent structures;
FIG.9 is a schematic sectional view of a sensor according to another embodiment of the present invention and the position relationship between the sensor and its adjacent structures.

### DETAILED DESCRIPTION

As mentioned above, the prior art body fluid parameter detection device has a greater thickness and is easy to get caught while being worn on the skin surface, which leads to poor user experiences and brings inconvenience to the patient's life.

After research, it is found that the reason for the above problem is that the height of the sensor structure protruding from the inner bottom surface of the bottom case in the prior detection device is large. Extra space is required along the thickness direction of the transmitter to accommodate the foresaid protruding portion, increasing the thickness of the transmitter.

In order to solve this problem, the present invention provides a micro-analyte detection device. The sensor base is completely embedded in the bottom case. Thus, the top of the sensor structure is not higher than or slightly higher than the inner bottom surface of the bottom case. Therefore, no extra space is required in the transmitter to accommodate the protruding portion, which reduces the thickness of the transmitter and enhances the user experience.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, the width, the length or the distance of certain units may be exaggerated relative to other structures.

The following description of the exemplary embodiments is merely illustrative, and is not intended to be in any way limiting the invention and its application or use. The techniques, methods and devices that are known to those of ordinary skill in the art may not be discussed in detail, but such techniques, methods and devices should be considered as part of the specification.

It should be noted that similar reference numerals and letters indicate similar items in the following figures. Therefore, once an item is defined or illustrated in a drawing, it will not be discussed further in following description of the drawings.

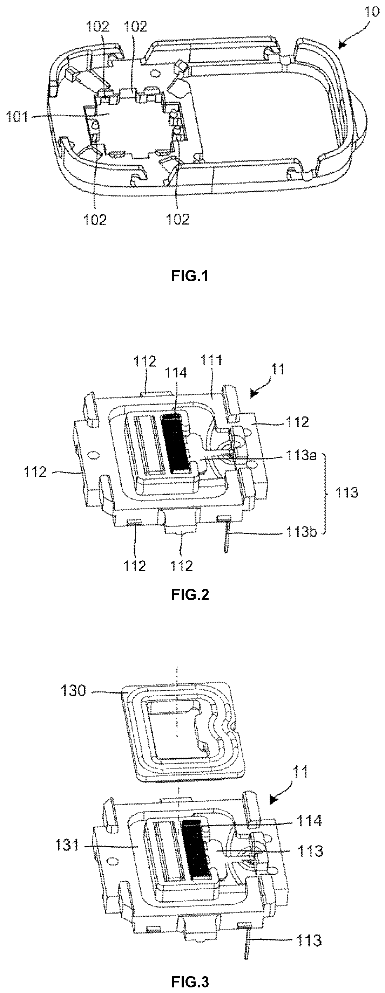

FIG.1 is a schematic perspective view of a bottom case 10 according to an embodiment of the present invention.

The bottom case 10 is used for mounting the sensor structure and the transmitter. In the embodiment of the present invention, the bottom board of the bottom case 10 is provided with a mounting hole 101 for mounting the sensor structure, while the fastener structure for fixing the transmitter is designed on the side wall of the bottom case 10.

In the embodiment of the present invention, a first fastener part 102 is provided at the edge of the mounting hole 101. Here, being provided at the "edge" means that the top of the first fastener part 102 is not higher than or slightly higher than the inner bottom surface of the bottom case 10.

The embodiment of the present invention does not specifically limit the shape and type of the first fastener part 102 and the shape of the mounting hole 101, as long as the conditions for mounting the sensor structure can be satisfied.

FIG.2 is a schematic perspective view of the sensor structure 11 according to an embodiment of the present invention.

The sensor structure 11 includes a sensor base 111 and a sensor 113. A second fastener part 112 is provided at the edge of the sensor base 111 for fastening with the first fastener part 102.

The sensor 113 includes a signal output end 113a and a detection end 113b. The signal output end 113a needs to be electrically connected with the electrical connection area of the transmitter to output the detection signal to the transmitter. The detection end 113b is used to pierce subcutaneous tissue to detect body fluid analyte parameters. In the embodiment of the present invention, the signal output end 113a is embedded in the sensor base 111.

FIG.3 is a schematic perspective view of the sealing member 130 and the sensor structure 11 according to the embodiment of the present invention.

The sealing member 130, which has dust-proof feature, moisture-proof feature, water-proof feature and the like, is configured to seal the connection position of the signal output end 113a and the electrical connection area.

The sensor base 111 of the embodiment of the present invention is provided with a groove 131 in which the sealing member 130 is placed. The installed sealing member 130 surrounds the connection position between the signal output end 113a and the electrical connection area to achieve sealing effect.

It should be noted that, in other embodiments of the present invention, when the transmitter is installed, the transmitter and the bottom case 10 have good sealing performance, therefore the sealing member 130 may not be provided.

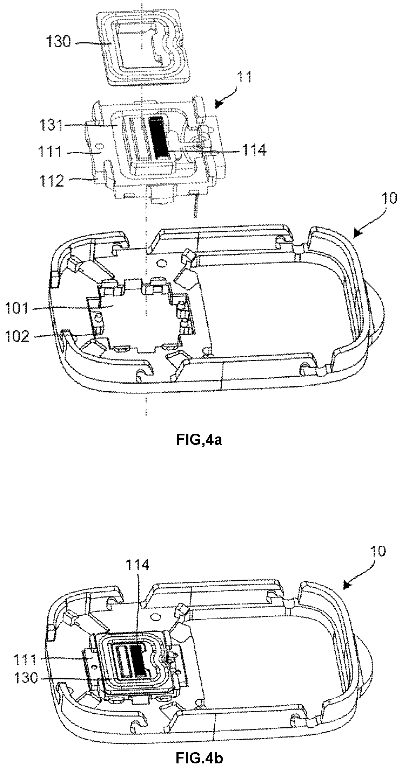

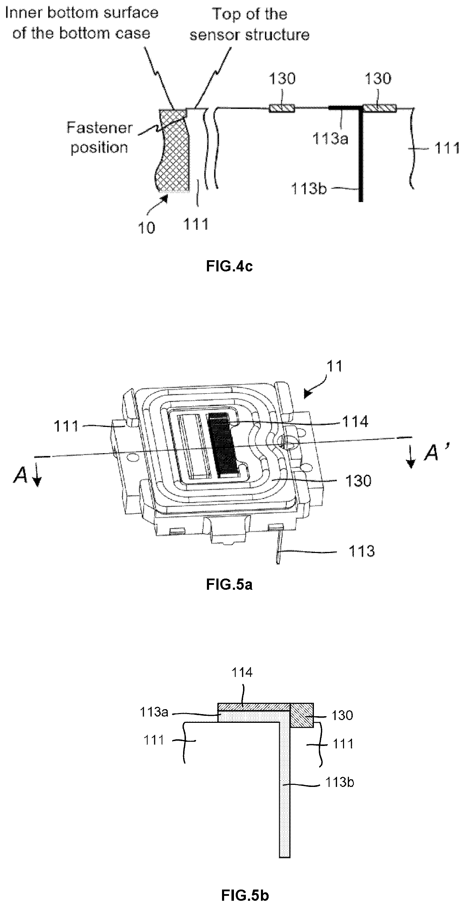

FIG.4a is a schematic perspective view of the bottom case 10, the sensor structure 11 and the sealing member 130 according to the embodiment of the present invention. FIG.4b is a schematic perspective view of the bottom case 10, the sensor structure 11 and the sealing member 130 according to the embodiment of the present invention. FIG.4c is a partial sectional view of the longitudinal section of FIG.4b.

As shown in FIG.4a, in the embodiment of the present invention, the bottom case 10, the sensor structure 11 and the sealing member 130 are assembled along the dashed line in proper order. As described above, the sealing member 130 will be installed in the groove 131 provided in the sensor base 111. And in the embodiment of the present invention, the contour shape of the edge of the probe base 111 matches the contour shape of the edge of the mounting hole 101. The second fastener part 112 at the edge of the sensor base 111 and the first fastener part 102 at the edge of the mounting hole 101 are fastened with each other. The edge of the sensor base 111 and the edge of the mounting hole 101 are interlocked with each other, and the sensor structure 11 is installed in its working position, as shown in FIG.4b.

In the embodiment of the present invention, the edge of the sensor base 111 and the edge of the mounting hole 101 are interlocked with each other, and the sensor base 111 is entirely embedded in the bottom board of the bottom case 10. And because the top of the first fastener part 102 is not higher than or slightly higher than the inner bottom surface of the bottom case 10, the top of the fastener position between the first fastener part 102 and the second fastener part 112 is also not higher than or slightly higher than the inner bottom surface of the bottom case 10, as shown in FIG.4c. Therefore, in the embodiment of the present invention, the distance between the top of the sensor structure 11 and the inner bottom surface of the bottom case 10 is less than or equal to 0.8 mm. In the embodiment of the present invention, after being mounted to the bottom case 10, the sensor base 111 is integrated with the bottom board of the bottom case 10 as a whole, and the sensor base 111 is completely embedded into the bottom board of the bottom case 10, which means the top of the sensor structure 11 and the inner bottom surface of the bottom case 10 are flush. In another embodiment of the present invention, after being mounted to the bottom case 10, the top of the sensor structure 11 is lower than the inner bottom surface of the bottom case 10. In another embodiment of the present invention, when the sensor structure 11 is installed in the working position, the top of the sensor structure 11 is slightly higher than the inner bottom surface of the bottom case 10.

In an existing body fluid analyte detection device, the fastener part is provided on one end of the sensor structure or on the side wall thereof, or other additional components are required to assist the fastening, so the fastener part on the bottom case should be set higher than the inner bottom surface of the bottom case. When the sensor structure is installed on the bottom case, the fastener position is higher than the inner bottom surface of the bottom case, which increases the distance between the top of the sensor structure and the inner bottom surface of the bottom case.

Compared with the existing body fluid analyte detection device, the sensor structure 11 of the analyte detection device according to the embodiment of the present invention has a much smaller height or zero height protruding from the inner bottom surface of the bottom case 10, or the top of the sensor structure 11 is lower than the inner bottom surface of the bottom case 10. Thus, no extra space is required along the thickness direction of the transmitter to accommodate the part of the sensor structure 11 protruding from the inner bottom surface of the bottom case 10, or only a little space with small height is required, which will greatly reduce the thickness of the transmitter and the overall thickness of the analyte detection device and improve the user experience.

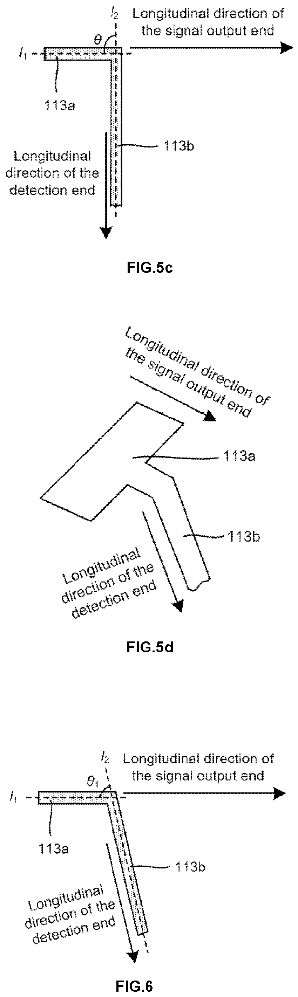

FIG.5a is a schematic perspective view of the sensor structure 11 after the sealing member 130 is installed. FIG.5b and FIG.5c are schematic sectional views of the longitudinal sections of the sensor 113 and its adjacent structures obtained along the section line A-A' in FIG.5a. FIG.5d is a schematic sectional view of a part of the sensor 113 according to the embodiment of the present invention.

In the embodiment of the present invention, since a groove 131 is provided on the sensor base 111 (as shown in FIG.4a), when the sealing member 130 is placed, the top of the sealing member 130 is flush with or slightly higher than the top of the sensor structure 11. For the sealing member 130 is generally elastic, even if the top of the sealing member 130 is slightly higher than the top of the sensor structure 11, it will not increase the thickness of the analyte detection device after the transmitter is installed.

As shown in FIG.5b and FIG.5c, the sensor 113's shape is a polyline. The straight line determined by the longitudinal direction of the signal output end 113a is *l*₁, and the straight line determined by the longitudinal direction of the detection end 113b is *l*₂, the angle between *l*₁ and *l*₂ is θ, θ ≠ 0°. In the embodiment of the present invention, *l*₁⊥*l*₂. *l*₂ is perpendicular to the skin surface of the puncture position, so *l*₁ is parallel to the skin surface of the puncture position. Compared with other possible positional relationships between *l*₁ and *l*₂, *l*₁⊥*l*₂ can optimize the design of the mounting equipment of the sensor structure 11, simplifying the sensor installation process for the user and facilitating the puncture process. Because the thickness of the sensor 113 is extremely thin (usually at micron level), the signal output end 113a hardly protrudes from the top of the sensor structure 11. Therefore, no extra space is required in the transmitter to accommodate the signal output end 113 that protrudes the inner bottom surface of the bottom case 111, which will ultimately reduce the overall thickness of the analyte detection device.

As shown in FIG.5d, the sensor 113 is T-shaped. The surface of the sensor 113 is provided with electrodes and circuits (not shown) for detecting the parameters of the analyte. The electrical signal from the detection end 113b is transmitted to the transmitter through the signal output end 113a. Therefore, the signal output end 113a is designed as a planar structure and is laid on the top of the sensor base 111 to facilitate electrical connection. In another embodiment of the present invention, the signal output end 113a is embedded in the sensor base 111, and the position to be connected with the electrical connection area of the transmitter is exposed.

In other embodiments of the present invention, the sensor 113 may also have other possible shapes, such as a structure with a curved surface, which is not specifically limited herein.

In another embodiment of the present invention, the sensor 113 is curved as a whole, that is, the sensor 113 has a shape of an arc, preventing the sensor 113 from being easily broken. In other embodiments of the present invention, the sensor 113 may also have the shape of a polyline with multiple straight or curved lines, or *l*₁ and *l*₂ can be in different planes, which is not specifically limited herein.

FIG.6 is a schematic sectional view of the longitudinal section of a part of a sensor 113 according to another embodiment of the present invention.

*θ*₁ < 90°. The signal output end 113a will hardly protrude from the top of the sensor structure 11 with a proper puncture direction of the sensor when the sensor structure 11 is installed. Therefore no extra space is required in the transmitter to accommodate the signal output end 113a, which further reduces the overall thickness of the analyte detection device.

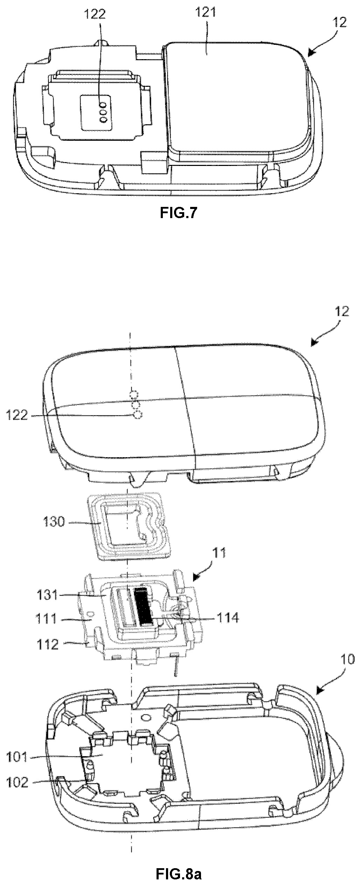

FIG.7 is a schematic perspective view of the transmitter 12 according to the embodiment of the present invention.

The transmitter 12 includes a transmitter housing 121 and an electronic device (not shown) provided inside the transmitter housing 121. The electrical connection area 122 of the electronic device is exposed and protrudes from the transmitter housing 121 to facilitate electrical connection with the signal output end 113a. As mentioned above, since the top of the sensor structure 11 does not protrude or protrudes slightly from the inner bottom surface of the bottom case 10, it is not necessary to provide extra space along the thickness direction of the transmitter 12, thus reducing the thickness of the transmitter.

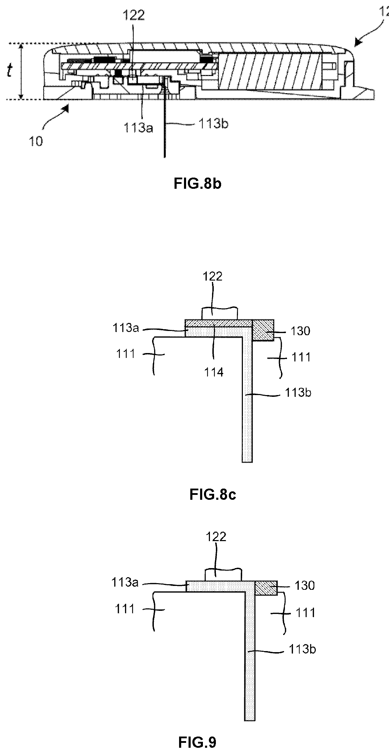

FIG.8a is a schematic perspective view of the bottom case 10, the sensor structure 11, the sealing member 130 and the transmitter 12 according to an embodiment of the present invention. FIG.8b is a side view of the micro-analyte detection device after installation according to an embodiment of the present invention. FIG.8c is a schematic view of the sensor 113 and its positional relationship with the adjacent structures in FIG.8b.

As shown in FIG.8a, the position of the electrical connection area 122, shown by the dotted circle, corresponds to the position of the signal output end 113a. When the transmitter 12 is installed in the bottom case 10, the electrical connection area 122 is electrically connected to the signal output end 113a to achieve signal transmission, as shown in FIG.8b and FIG.8c.

In the embodiment of the present invention, a conductive rubber strip 114, which is elastic, is provided between the electrical connection region 122 and the signal output end 113a. After the transmitter is installed, the electrical connection area 122 is electrically connected to the signal output end 113a by pressing the conductive rubber strip 114, which ensures good electrical connection.

As mentioned above, in the existing body fluid analyte detection device, the fastener position between the sensor structure and the bottom case is higher than the inner bottom surface of the bottom case; or *θ* = 0 °, that is, the entire sensor is perpendicular to the skin surface of the puncture position. The distance between the top of the sensor structure and the inner bottom surface of the bottom case includes not only the height of the fastener position but also the length of the signal output end. Therefore, the distance between the top of the sensor structure and the bottom surface of the bottom case is generally large. Thus, extra space must be provided in the transmitter to accommodate the parts protruding from the bottom board of the bottom case, resulting in thicker analyte detection device and poorer user experience.

If the overall thickness of the analyte detection device is t, compared with the existing detection device, the thickness t of the analyte detection device in the embodiment of the present invention will be reduced by more than 35%, as shown in FIG.8b, which greatly enhances user experience.

FIG.9 is a schematic sectional view of a sensor 113 and its position relationship with adjacent structures according to another embodiment of the present invention.

This embodiment of the present invention is not provided with a conductive rubber strip, and the electrical connection area 122 is directly in electrical contact with the signal output end 113a, which further reduces the overall thickness of the analyte detection device.

In summary, the present invention relates to a micro-analyte detection device. Because the edge contour shapes of both the probe structure and the mounting hole match each other, after the two are fastened with each other, the sensor base is entirely embedded in the bottom board of the bottom case, which reduces the distance between the inner bottom surface and the top of the sensor structure. There is no need to provide extra space in the transmitter, which ultimately reduces the thickness of the analyte detection device and enhances the user experience.

## Claims

1. A micro-analyte detection device, **characterized in that**, including:
a bottom case, a bottom board of the bottom case is provided with a mounting hole, and a first fastener part is provided at an edge of the mounting hole;
a sensor structure, the sensor structure includes a sensor and a sensor base, and the sensor includes a signal output end and a detection end, a second fastener part is provided at an edge of the sensor base, contour shapes of the edge of the sensor base and of the mounting hole match each other, the first fastener part and the second fastener part are fastened with each other, the edge of the mounting hole and the edge of the sensor base are interlocked, and the sensor base is installed in the mounting hole; and
a transmitter, the transmitter includes a transmitter housing and an electronic device disposed in the transmitter housing, the electronic device is provided with an electrical connection area, when the transmitter is installed in a working position, the electrical connection area is electrically connected with the signal output end,
wherein the sensor's shape is an arc or a polyline,
wherein a straight line determined by a longitudinal direction of the signal output end is perpendicular to a straight line determined by a longitudinal direction of the detection end.

2. The micro-analyte detection device of claim 1, **characterized in that**,
the signal output end is a planar structure and is laid on a top of the sensor base.

3. The micro-analyte detection device of claim 1, **characterized in that**,
the straight line determined by the longitudinal direction of the detection end is perpendicular to a skin surface where the detection end punctures.

4. The micro-analyte detection device of claim 1, **characterized in that**,
the electrical connection area is exposed outside the transmitter housing, and a position of the electrical connection area corresponds to a position of the signal output end.

5. The micro-analyte detection device of claim 1, **characterized in that**,
further includes a conductive rubber strip, which is disposed between the electrical connection area and the signal output end, and the electrical connection area and the signal output end are electrically connected through the conductive rubber strip.

6. The micro-analyte detection device of claim 1, **characterized in that**,
further includes a sealing member, and the sealing member is arranged around a position where the signal output end is electrically connected with the electrical connection area.

## Patentansprüche

1. Mikroanalyt-Nachweisvorrichtung, **dadurch gekennzeichnet, dass** es enthält:
ein unteres Gehäuse, wobei eine Bodenplatte des unteren Gehäuses mit einem Montageloch bereitgestellt ist und ein erstes Befestigungsteil an einer Kante des Montagelochs bereitgestellt wird;
eine Sensorstruktur, wobei die Sensorstruktur einen Sensor und eine Sensorbasis enthält und der Sensor ein Signalausgangsende und ein Erfassungsende enthält, ein zweites Befestigungsteil an einer Kante der Sensorbasis bereitgestellt ist, Konturformen der Kante der Sensorbasis und des Montagelochs zueinander passen, wobei das erste Befestigungsteil und das zweite Befestigungsmittelteil aneinander befestigt sind, die Kante des Montagelochs und die Kante der Sensorbasis ineinander greifen und die Sensorbasis in dem Montageloch montiert ist; und
ein Sender, wobei der Sender ein Sendergehäuse und eine elektronische Vorrichtung enthält, die in dem Sendergehäuse angeordnet ist, wobei die elektronische Vorrichtung mit einem elektrischen Anschlussbereich bereitgestellt wird, wobei der elektrische Anschlussbereich elektrisch mit dem Signalausgangsende verbunden ist, wenn der Sender in einer Arbeitsposition installiert ist,
wobei die Form des Sensors ein Bogen oder eine Polylinie ist,
wobei eine gerade Linie, die durch eine Längsrichtung des Signalausgabeendes bestimmt wird, senkrecht zu einer geraden Linie ist, die durch eine Längsrichtung des Erfassungsendes bestimmt wird.

2. Mikroanalyt-Erfassungsvorrichtung gemäß Anspruch 1, **dadurch gekennzeichnet, dass** das Signalausgabeende eine planare Struktur ist und auf einer Oberseite der Sensorbasis liegt.

3. Mikroanalyt-Erfassungsvorrichtung gemäß Anspruch 1, **dadurch gekennzeichnet, dass** die gerade Linie, die durch die Längsrichtung des Erfassungsendes bestimmt ist, senkrecht zu einer Hautoberfläche ist, in die das Erfassungsende eindringt.

4. Mikroanalyt- Erfassungsvorrichtung gemäß Anspruch 1, **dadurch gekennzeichnet, dass** der elektrische Anschlussbereich außerhalb des Sendergehäuses freiliegt und eine Position des elektrischen Anschlussbereichs einer Position des Signalausgabeendes entspricht.

5. Mikroanalyt- Erfassungsvorrichtung gemäß Anspruch 1, **dadurch gekennzeichnet, dass** sie ferner einen leitfähigen Gummistreifen enthält, der zwischen dem elektrischen Anschlussbereich und dem Signalausgangsende angeordnet ist, und der elektrische Anschlussbereich und das Signalausgangsende durch den leitfähigen Gummistreifen elektrisch verbunden sind.

6. Mikroanalyt- Erfassungsvorrichtung gemäß Anspruch 1, **dadurch gekennzeichnet, dass** sie ferner ein Dichtungselement enthält und das Dichtungselement um eine Position herum angeordnet ist, an der das Signalausgangsende elektrisch mit dem elektrischen Anschlussbereich verbunden ist.

## Revendications

1. Dispositif de détection de micro-analyte, **caractérisé en ce que**, comprenant :
un casier de partie inférieure, une planche de partie inférieure du casier de partie inférieure est fournie avec un trou de montage et une première partie d'attache est fournie à un bord du trou de montage ;
une structure de capteur, la structure de capteur comprend un capteur et une base de capteur et le capteur comprend une extrémité de sortie de signal et une extrémité de détection, une seconde attache est fournie un bord de la base de capteur les formes de contour du bord de la base de capteur et du trou de montage s'apparient l'un à l'autre, la première partie d'attache et la seconde partie d'attache sont attachées l'une avec l'autre, le bord du trou de montage et le bord de la base de capteur sont interverrouillés et la base de capteur est installée dans le trou de montage ; et
un transmetteur, le transmetteur comprend un logement de transmetteur et un dispositif électronique disposé dans le logement de transmetteur, le dispositif électronique est fourni avec une zone de connexion électrique, quand le transmetteur est installé dans une position de travail, la zone de connexion électrique est connectée électriquement avec l'extrémité de sortie de signal,
la forme du capteur étant un arc ou une polyligne,
une ligne droite déterminée par une direction longitudinale de l'extrémité de sortie de signal étant perpendiculaire à une ligne droite déterminée par une direction longitudinale de l'extrémité de détection.

2. Dispositif de détection de micro-analyte selon la revendication 1, **caractérisé en ce que**, l'extrémité de sortie de signal est une structure planaire et est posée sur une partie supérieure de la base de capteur.

3. Dispositif de détection de micro-analyte selon la revendication 1, **caractérisé en ce que**,
la ligne droite déterminée par la direction longitudinale de l'extrémité de détection est perpendiculaire à une surface de peau où l'extrémité de détection est percée.

4. Dispositif de détection de micro-analyte selon la revendication 1, **caractérisé en ce que**,
la zone de connexion électrique est exposée à l'extérieur du logement de transmetteur et une position de la zone de connexion électrique correspond à une position de l'extrémité de sortie de signal.

5. Dispositif de détection de micro analyte selon la revendication 1, **caractérisé en ce que**,
il comprend en outre une bande de caoutchouc conductrice, qui est disposée entre la zone de connexion électrique et l'extrémité de sortie de signal et la zone de connexion électrique et l'extrémité de sortie de signal sont connectées électriquement par la bande de caoutchouc conductrice.

6. Dispositif de détection de micro-analyte selon la revendication 1, **caractérisé en ce que**,
il comprend en outre un élément de scellement et l'élément de scellement est disposé autour d'une position où l'extrémité de sortie de signal est connectée électriquement avec la zone de connexion électrique.
